# EUROPEAN PATENT APPLICATION

(11) **EP 1 074 222 A2**
(43) Date of publication of application: **07.02.2001**
(21) Application number: 00500175.5
(22) Date of filing: 31.07.2000
(51) Int. Cl.: A61B 6/00

(54) **Device for the injection of co2 for angiography**

(30) Priority: 04.08.1999 ES 9901774
(71) Applicant: Grupo Grifols, S.A., 08150 Parets Del Valles, Barcelona (ES)
(72) Inventor: Martinell Gisper-Sauch, Enrique, 08010 Barcelona (ES); Ferrer Royo, Roger, 08190 Sant Cugat del Vallés, Barcelona (ES); Manzano Riera, Jorge, 08009 Barcelona (ES)
(74) Representative: Duran Moya, Luis-Alfonso

(57) **Abstract**

The device comprises a unit constituted by a plunger pump which meters CO₂ and which is connected to a pressure transducer of the said gas which is in turn connected to a system of two valves integrated in a single support body and connected to one another by a single duct, each of them being regulated by a three-way valve and being intended, respectively, for the control of the CO₂ and for the control of a physiological saline solution, the body of the valves having an outlet connection for the coupling thereof to a catheter for the administration of CO₂ for angiography.

## Description

The present invention relates to a device which is intended especially for the metered injection of CO₂ for application in angiography, providing a large number of advantages over the devices currently known for this function.

As is known, the injection of CO₂ for angiography has been introduced with notable success. The syringes currently used for the purpose are of a more or less conventional type and, once the carbonic anhydride has been drawn into their interior, they subsequently enable the doctor to introduce the desired amount of CO₂ by manual pressure, by means of the syringe, and to control the contrast effect produced.

The object of the present invention is to provide means enabling the supply of CO₂ for angiography to be effected under mechanised control conditions by an item of equipment intended for single use which also carries means which are efficient in the filtering of gas and in the control of the entry of a saline solution.

In order to achieve its objects, the device to which the present invention relates comprises a pump having a calibrated cylinder and a plunger which is displaceable manually or in a mechanised manner, which pump is connected by a rapid lock of the luer type to an electronic pressure transducer likewise connected by a luer lock system to a set of two valves in series, with a longitudinal connecting duct, one of the valves being intended for the intake of CO₂ and the second being intended for the perfusion of a physiological solution. A high-pressure flexible duct having a luer lock connection of a rotatory type at both ends permits connection to the catheter for the introduction of CO₂ into the patient's circulatory system. At the CO₂ inlet, the device has a high-efficiency 0.22-micron filter.

Although the device can be operated manually, its constitution is expressly intended for mechanised operation by means of a motor for controlling the position of the plunger of the CO₂-metering pump and a respective motor for operating each of the control valves of the CO₂ circuit and of the physiological saline solution circuit. The electronic transducer is to control the CO₂ pressure continuously throughout the procedure, providing a signal which can be presented in a suitable form for its external display, and monitoring the pressure of the gas in the metering pump before and during injection and also any drop in CO₂ pressure in the circuit, possibly caused by gas leaks.

The valves carry control brackets arranged in an inverted position in order to permit their motorised operation by cups of matching form which carry the corresponding stops for limiting the displacement of the valves and preventing incorrect rotation positions.

The use of the device to which the present invention relates provides increased safety in the process of angiography by CO₂, both from the point of view of hygiene and from the electrical and mechanical point of view, thus providing for safer exploration and reducing the possibility of failure. From the point of view of hygiene, the constitution of the device as a whole as a single-use device has hygienic features hitherto unknown in this technique. Electrical safety includes protection against electric shocks, with levels of leakage currents to patients suitable for the cardiac use thereof, and safety under conditions of simple failure of the components.

Some drawings corresponding to a preferred embodiment of the invention are appended by way of nonlimiting example for a better understanding thereof.

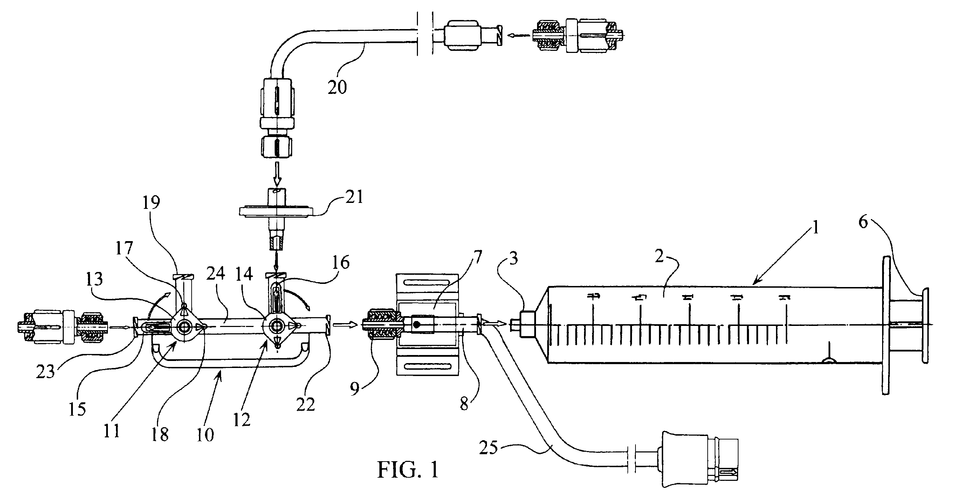

Figure 1 is a plan view of the device as a whole.

Figures 2 and 3 are both sectional details of the metering pump.

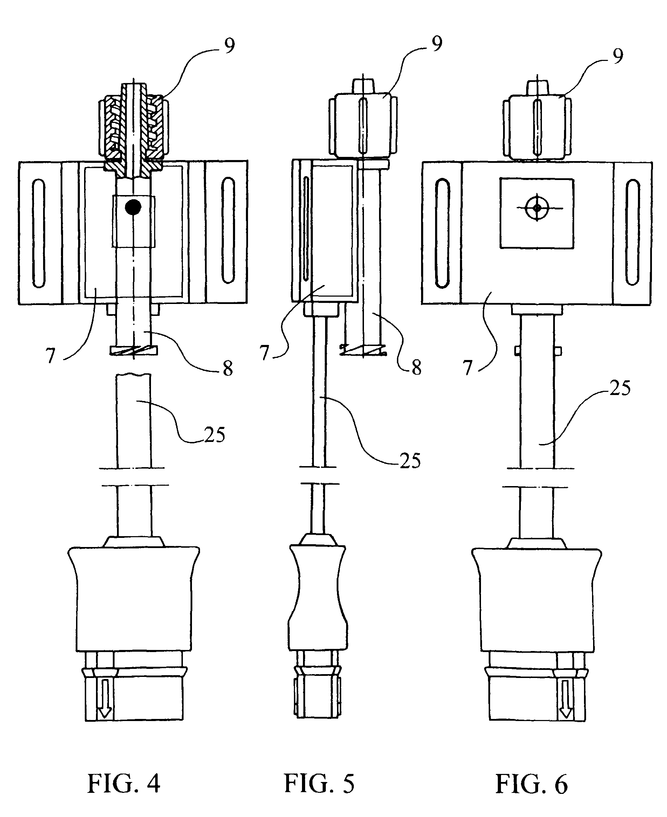

Figures 4, 5 and 6 are dihedral views of the transducer unit with its fluid connections and its electrical connection.

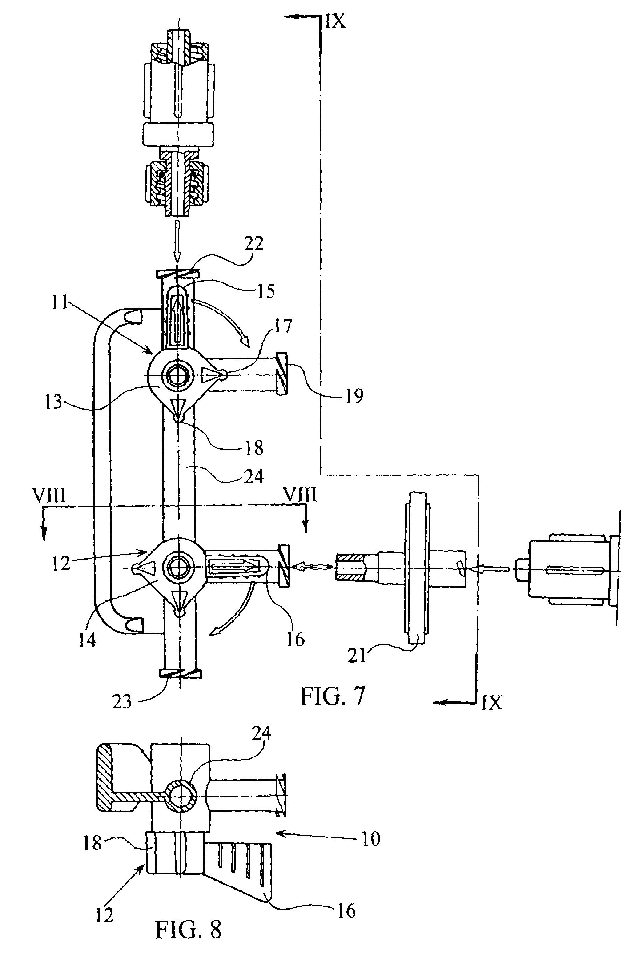

Figure 7 shows its valve unit with the accessories thereof disassembled for better observation.

Figure 8 is a section along the plane shown in Figure 7.

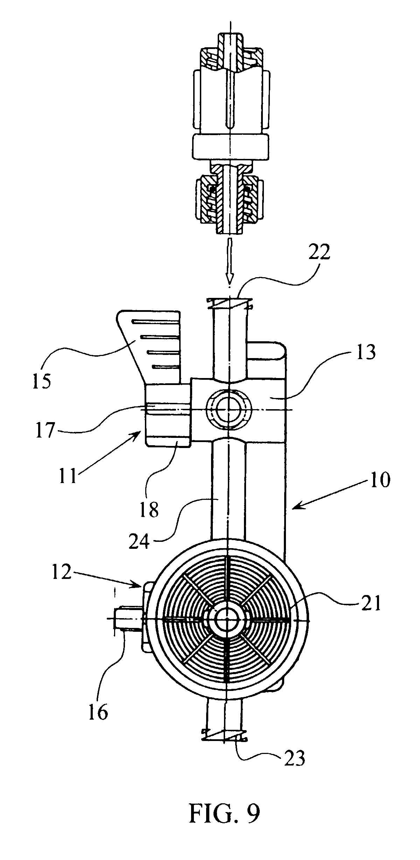

Figure 9 is a detail of the valve units of the device.

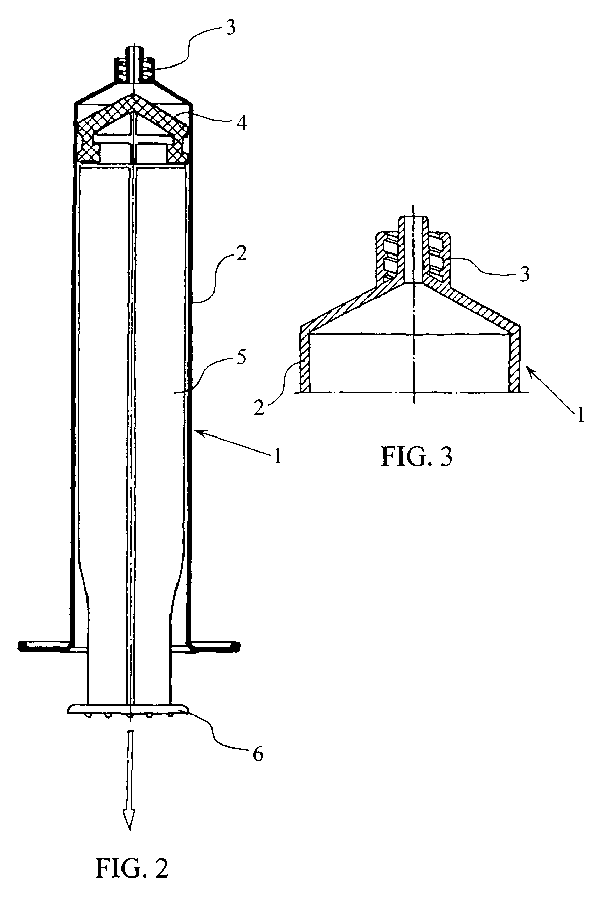

In accordance with the drawings, the single-use device to which the present invention relates and which is in the form of a sterile unit, basically comprises a CO₂-metering pump 1 formed by a calibrated cylinder 2 having a luer lock connection terminal 3 and an inner plunger 4, Figure 2, coupled to the operating rod 5 which has, externally, an extension 6 for both manual and, preferably, mechanised operation, the said unit being associated with the transducer 7 which is coupled by way of its connecting nozzle 8 to the luer lock outlet of the metering pump unit 1 and, by way of an outlet duct and the corresponding luer lock connection 9, to the valve unit 10. With this arrangement, the transducer controls the CO₂ pressure throughout the entire procedure, sending a corresponding electrical signal by way of the conductor 25 to the control apparatus, which enables the gas pressure and its variations, and also any drop in pressure resulting from leaks or incorrect connections or other defects, to be monitored.

The valve unit 10 comprises two valves 11 and 12 which, respectively, are to control the entry of a saline solution and to control the CO₂ gas. Each of them has a corresponding operating switch 13 and 14, with their corresponding driving brackets 15 and 16. Arranged on the brackets and on the bodies of the switches 13 and 14 are indicators, such as the projections represented on the switch 13 with the numerals 17 and 18, for indicating as a whole the communication situation of the three passages controlled by the valve.

Likewise, the valve unit 10 has a terminal 22 for coupling it to the luer lock connection 9 of the pressure transducer and another terminal at the opposite end 23 for connecting it, likewise by way of a luer lock device, to the catheter for administration to the patient. In the body 10 itself is an intermediate duct 24 for communication between the terminals 22 and 23, forming a single flow unit which includes the three-way valves 11 and 12.

The valve 11 is to control the entry of physiological saline solution which comes from the inlet 19. The valve 12 is to control the passage of CO₂, which it receives from the high-pressure resilient connection 20, through a 0.22-µ filter 21 and the corresponding intermediate connections.

By means of the explained constitution of the device to which the present invention relates, it is possible to effect the controlled metering of CO₂ for angiography under conditions of great asepsis and safety. The device, which is principally intended for mechanised use with motors which control the position of the plunger 5 of the metering pump and also the position of the switches 13 and 14 of the valves 11 and 12, could also be used manually, being especially designed for single use.

The description has been given with reference to CO₂ as the contrast gas and saline solution as the irrigation fluid. However, the present invention could be applied using any other gas usable as a contrast medium for angiography and any other irrigation fluid also usable for this technique.

## Claims

1. Device for the injection of CO₂ for angiography, characterised in that it comprises a unit constituted by a plunger pump which meters CO₂ and which is connected to a pressure transducer of the said gas which is in turn connected to a system of two valves integrated in a single support body and connected to one another by a single duct, each of them being regulated by a three-way valve and being intended, respectively, for the control of the CO₂ and for the control of a physiological saline solution, the body of the valves having an outlet connection for the coupling thereof to a catheter for the administration of CO₂ for angiography.

2. Device for the injection of CO₂ in angiography, according to claim 1, characterised in that the pneumatic pump for metering CO₂ is constituted by a syringe provided with a luer lock outlet connection and a plunger operable by a motor.

3. Device for the injection of CO₂ in angiography, according to claim 1, characterised in that each of the valves has a three-position switch with a bracket indicating one of the positions and projecting bosses indicating the other two positions, being capable of mechanised operation by means of matching cups of the switches.

4. Device for the injection of CO₂ in angiography, according to claim 1, characterised in that the CO₂ control valve is connected to the gas supply source through a high-pressure flexible tube and a 0.22-µ filter with connections of the luer lock type.
